# NEW EUROPEAN PATENT SPECIFICATION

(11) **EP 2 474 296 B2**
(45) Date of publication and mention of the opposition decision: **25.09.2019**
(45) Mention of the grant of the patent: 02.11.2016
(21) Application number: 10813759.7
(22) Date of filing: 02.09.2010
(51) Int. Cl.: A61K 8/06, A61K 8/34, A61K 8/37, A61K 8/891, A61Q 1/10, A61Q 1/12, A61Q 5/00, A61Q 17/04, A61Q 19/00, A61Q 19/10, B01J 13/00

(54) **METHOD FOR PRODUCING O/W EMULSION COMPOSITION**
VERFAHREN ZUR HERSTELLUNG EINER ÖL-IN-WASSER-EMULSIONSZUSAMMENSETZUNG
MÉTHODE DE PRODUCTION D'UNE COMPOSITION D'ÉMULSION HUILE DANS EAU

(30) Priority: 04.09.2009 JP 2009205236
(43) Date of publication of application: 11.07.2012
(73) Proprietor: Shiseido Company, Ltd., Tokyo 104-0061 (JP)
(72) Inventor: ARAKI Hidefumi, Yokohama-shi Kanagawa 224-8558 (JP); MIYAHARA Reiji, Yokohama-shi Kanagawa 224-8558 (JP)
(74) Representative: Henkel, Breuer & Partner
(86) International application number: PCT/JP2010/065003
(87) International publication number: WO 2011/027811

(56) References cited:
- WO-A1-2010/082602
- JP-A- 1 155 941
- JP-A- 60 125 244
- JP-A- 61 153 134
- US-A1- 2006 032 001
- US-A1- 2007 299 121

## Description

### FIELD OF THE INVENTION

The present invention relates to a production method of an O/W emulsion composition, and in particular, relates to a production method that is excellent in simplicity and economy.

### BACKGROUND OF THE INVENTION

In the past, in order to maintain the emulsion stability of external skin preparations such as cosmetics, quasi-drugs, and pharmaceuticals, an O/W emulsion composition with the use of an α-gel, which is formed from a higher aliphatic alcohol and a polyoxyethylene-type nonionic surfactant, has been used and it is especially known for use in cosmetics. As the preparation method of such an O/W emulsion composition, the following method has been used: a moisturizer and a hydrophilic polyoxyethylene-type nonionic surfactant are dissolved in water and heated to about 70 °C to prepare a water phase; an oil component and a higher alcohol, as essential components, were homogeneously mixed at about 70 °C to prepare an oil phase; the oil phase was added and emulsified into the water phase while being stirred with a homogenizer; and the obtained emulsion is rapidly cooled with a cooling machine such as an Onlator to about 35 °C (for example, refer to Non-patent Literature 1).

However, the conventional method, wherein the cooling is carried out after emulsification at about 70 °C, is wasteful because of the energy necessary for heating and for the use of a heat exchanger. In addition, the environmental burden is high because a large amount of water is used for washing a cooling machine such as an Onlator after use.

Accordingly, the development of an O/W emulsion composition that has comparable usability to the conventional one and can be economically and easily produced without using a conventional cooling apparatus such as an Onlator has been awaited.

Patent Literature 1 discloses 2-(amino or substituted amino)-5,6-substituted phenol compounds, compositions comprising such compounds, and the use of such compositions for oxidative dyeing of keratin fibers. In this context, a process for producing an O/W emulsion is described, comprising the steps of (i) emulsifying, at 70 to 75 °C, an oil phase comprising Steareth-21, Oleth-2, Oleth-5, PEG-50 and oleyl alcohol with a portion of water to prepare a high-temperature emulsified part that is an O/W emulsion, (ii) mixing the remaining water phase, which is 40 mass% of the total amount of water employed, with the high-temperature emulsified part while the high-temperature emulsified part is being stirred, to provide a mixture, (iii) cooiing the mixture to 35 °C, and (iv) stopping the stirring.

Non-patent Literature 2 discloses a low-energy emulsification method for preparing a non-ionic O/W emulsion composition.

### RELATED ART DOCUMENTS

### NON-PATENT LITERATURE

Non-patent Literature 1: "Chemistry and Application of Surface Activity" (in Japanese) written by Manabu Senoo, Dainippon Tosho Co., Ltd., 1995; p 160.
Non-patent Literature 2: Lin et al., J. Soc. Cosmet. Chem. 1978 (29), 745-756

### PATENT LITERATURE

Patent Literature 1: United States Patent Application Publication No. 2006/0032001 A1

### SUMMARY OF THE INVENTION

### PROBLEM TO BE SOLVED BY THE INVENTION

The present invention was made in view of the above-described situation of the background art, and the problem to be solved is to provide a simple and economical production method of an O/W emulsion composition.

### MEANS TO SOLVE THE PROBLEM

In view of the above-described problems, the present inventors have diligently studied; as a result, the present inventors have found that an O/W emulsion composition having the usability comparable to the conventional one can be produced without using the conventional preparation method with using in which an Onlator is used. The O/W emulsion composition can be produced by: emulsifying an oil phase comprising specific components (such as a nonionic surfactant, a linear higher alcohol that has 16 or more carbon atoms and can form an α-gel in water with the nonionic surfactant, and an oil component), which have a melting point at room temperature or higher, with only a portion of water phase components (such as water and moisturizer) at 70 °C or higher; mixing the obtained highly-concentrated O/W emulsified part with the remaining low-temperature water phase (main water phase) to cool the emulsified part with continuous stirring until the lower temperature limit, or lower, of the temperature range wherein the oil phase forms an α-gel in the water phase is reached; and then stopping the stirring. This finding has led to completion of the present invention.

In one aspect, the invention thus relates to a production method of an O/W emulsion composition as defined in claim 1. The method comprises the steps of:
emulsifying, at 70 °C or higher, an oil phase with a portion of a water phase to prepare a high-temperature emulsified part that is a highly concentrated O/W emulsion,
   wherein
   the oil phase comprises
   (A) a nonionic surfactant,
   (B) a linear higher alcohol that has 16 or more carbon atoms and can form an α-gel in water with the nonionic surfactant, and
   (C) an oil component, and
      the water phase comprises
   (D) water;
mixing the remaining water phase, which is 50 to 75 mass% of the total amount of the O/W emulsion composition and at 10 to 35 °C, with the high-temperature emulsified part while the high-temperature emulsified part is being stirred, to rapidly cool the high-temperature emulsified part with continuous stirring to the lower temperature limit, or lower, of a temperature range wherein the oil phase forms an α-gel in the water phase, wherein the lower temperature limit of the temperature range wherein the oil phase forms the α-gel in the water phase is 4 °C lower than the peak temperature of the exothermic peak, which is measured by differential scanning calorimetry DSC, of the high-temperature emulsified part; and
then stopping the stirring.

In another aspect, the invention relates to a production method of a O/W emulsion composition as defined in claim 2. The O/W emulsion composition comprises a liquid oil component and the method comprises the steps of:
emulsifying, at 70 °C or higher, an oil phase with a portion of a wafer phase to prepare a high-temperature emulsified part that is a highly concentrated O/W emulsion, wherein
   the oil phase comprises
   (A) a nonionic surfactant,
   (B) a linear higher alcohol that has 16 or more carbon atoms and can form an α-gel in water with the nonionic surfactant, and
   (C) an oil component, and
      the water phase comprises
   (D) water; wherein at least a portion of the liquid oil component is not contained in the oil phase;
mixing the remaining water phase, which is 50 to 75 mass% of the total amount of the O/W emulsion composition and at 10 to 35 °C, with the high-temperature emulsified part while the high-temperature emulsified part is being stirred, to rapidly cool the high-temperature emulsified part with continuous stirring to the lower temperature limit, or lower, of a temperature range wherein the oil phase forms an α-gel in the water phase;
adding the remaining liquid oil component to the mixture at the lower temperature limit, or lower, of the α-gel formation temperature range; and
then stopping the stirring.

It is preferable in the production method of the O/W emulsion composition that the liquid oil component in the oil phase for the preparation of the high-temperature emulsified part is less than 5 mass % of the total amount of the liquid oil component in the O/W emulsion composition.

It is preferable in the production method of the O/W emulsion composition that the lower temperature limit of the temperature range wherein the oil phase forms the α-gel in the water phase is 8 °C lower than the peak temperature of the exothermic peak, which is measured by differential scanning calorimetry DSC, of the high-temperature emulsified part.

### EFFECT OF THE INVENTION

According to the production method of the O/W emulsion composition of the present invention, an O/W emulsion composition can be easily produced with low energy without using a cooling machine such as an Onlator; thus it is very economical. In addition, the O/W emulsion composition produced by the production method of the present invention has comparable quality and usability to those of the O/W emulsion composition that has the same formulation and is produced with the use of a cooling machine such as an Onlator.

### BRIEF DESCRIPTION OF THE DRAWINGS

Fig 1 shows a result for the DSC measurement of an emulsified part for an O/W emulsion composition of the present invention.
Fig 2 shows a result for the DSC measurement of an emulsified part for an O/W emulsion composition of the present invention.

### MODE FOR CARRYING OUT THE INVENTION

Hereinafter, the constitution of the present invention will be described in detail. The O/W emulsion composition can be obtained by the method of claim 1:
emulsifying, at 70 °C or higher, an oil phase with a portion of a water phase to prepare a high-temperature emulsified part that is a highly concentrated O/W emulsion, wherein the oil phase comprises (A) a nonionic surfactant, (B) a linear higher alcohol that has 16 or more carbon atoms and can form an α-gel in water with the nonionic surfactant, and (C) an oil component, and the water phase comprises (D) water;
mixing the remaining water phase, which is 50 to 75 mass% of the total amount of the O/W emulsion composition and at 10 to 35 °C, with the high-temperature emulsified part while the high-temperature emulsified part is being stirred, to rapidly cool the high-temperature emulsified part to the lower temperature limit, or lower, of a temperature range wherein the oil phase forms an α-gel in the water phase, and
continuing the stirring until the temperature becomes the lower temperature limit, or lower, of the α-gel formation temperature range. The O/W emulsion composition is characterized in that an α-gel is formed in the interface of the emulsion particles of the O/W emulsion composition.

When the blending of a liquid oil component to the O/W emulsion composition of the present invention is desired, the O/W emulsion composition can also be obtained by preparing the emulsified part with the use of the oil phase from which at least a portion of the liquid oil component is excluded, cooling the emulsified part to the lower temperature limit, or lower, of the α-gel formation temperature range with the mixing of the main water phase, and then adding the remaining liquid oil components to the obtained mixture followed by stopping the stirring.

The α-gel is an associated complex consisting of lamellar bimolecular membranes, which are formed by a surfactant with a linear higher alcohol having 16 or more carbon atoms or a non-neutralized fatty acid in the presence of water. This α-gel formation temperature varies depending upon the chain length of the higher alcohol or fatty acid and the mole ratio between the higher alcohol or non-neutralized fatty acid and the surfactant. This is described in "Physical Chemistry of Cetyl Alcohol" (in Japanese) (Fragrance Journal Ltd., 1992) written by Shoji Fukushima.
(A) A nonionic surfactant used in the present invention is not limited in particular so far as the oil phase can form an α-gel, in the water phase, with the nonionic surfactant. Examples of (A) the nonionic surfactant used in the present invention include polyoxyethylene glycerol fatty acid esters, polyoxyethylene methylpolysiloxane copolymer, polyoxyethylene sorbitan fatty acid esters, polyoxyethylene alkyl ethers, maltitol hydroxy aliphatic alkyl ethers, alkylated polysaccharides, alkyl glucosides, sucrose fatty acid esters, and polyoxyethylene hydrogenated castor oil glyceryl. Hydrophilic surfactants are preferable, and in particular, those with the HLB of 8 or higher are preferable. However, a lipophilic nonionic surfactant such as glyceryl monostearate can be used in combination therewith.
(B) A linear higher alcohol having 16 or more carbon atoms used in the present invention is not limited in particular so far as it can form an α-gel with a nonionic surfactant in water and it can be used in the fields of cosmetics, pharmaceuticals, quasi-drugs, etc. Examples thereof include cetyl alcohol, stearyl alcohol, and behenyl alcohol. Preferably, it is a linear higher alcohol having 16 to 24 carbon atoms.
   For the formation of α-gel, it is preferable to add batyl alcohol, monoglycerides, etc.
   The concentrations of (A) a nonionic surfactant and (B) a linear higher alcohol having 16 or more carbon atoms are not limited in particular. Preferably, the total amount of (A) the nonionic surfactant and (B) the linear higher alcohol having 16 or more carbon atoms, in the O/W emulsion composition, is 0.5 to 10 parts by mass with respect to 10 parts by mass of (C) the oil component. If the total amount is less than 0.5 parts by mass, a highly stable O/W emulsion composition may not be obtained because of low surfactant content. If the total amount exceeds 10 parts by mass, there is an unfavorable trend from the standpoint of usability because of too much surfactant.
(C) An oil component used in the present invention is not limited in particular. In order to adjust the α-gel formation temperature range, solid oil components and liquid oil components can be separately used for the mixing and addition.

The solid oil components in the present invention include oil components that are normally used in cosmetics and external skin preparations and are solid at room temperature. Specific examples thereof include solid fats such as cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neat's-foot oil, Japan wax, and hydrogenated castor oil; waxes such as beeswax, candelilla wax, cotton wax, carnauba wax, bayberry wax, ibota wax, spermaceti wax, montan wax, rice bran wax, lanolin, kapok wax, acetylated lanolin, sugar cane wax, lanolin fatty acid isopropyl ester, hexyl laurate, hydrogenated lanolin, jojoba wax, lanolin wax, shellac wax, POE lanolin alcohol ether, POE lanolin alcohol acetate, POE cholesterol ether, and POE hydrogenated lanolin alcohol ether; hydrocarbon waxes such as polyethylene wax, paraffin wax, ceresin, petrolatum, microcrystalline wax, Lunacera, and ozokerite; fatty acid glyceryl ethers such as monostearyl glycerin ether (batyl alcohol); fatty acid glycerides such as acetoglyceride and tri-2-heptylundecanoic acid glyceride. These solid oil components can be blended either alone or in combination of two or more.

The liquid oil components used in the present invention include oil components that are normally used in cosmetics and external skin preparations and are liquid at room temperature. Specific examples thereof include liquid fats such as avocado oil, evening primrose oil, camellia oil, turtle oil, macadamia nut oil, sunflower seed oil, almond oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, china paulownia oil, Japanese paulownia oil, jojoba oil, and germ oil; ester oils such as cetyl octanoate, cetyl 2-ethylhexanoate, hexyldecyl dimethyloctanoate, ethyl laurate, hexyl laurate, isopropyl myristate, 2-hexyldecyl myristate, myristyl myristate, octyldodecyl myristate, isopropyl palmitate, 2-ethylhexyl palmitate, 2-hexyldecyl palmitate, 2-heptylundecyl palmitate, butyl stearate, isocetyl stearate, isocetyl isostearate, decyl oleate, dodecyl oleate, oleyl oleate, myristyl lactate, cetyl lactate, diisostearyl malate, cholesteryl 12-hydroxystearate, castor oil fatty acid methyl ester, 2-octyldodecyl N-lauroyl-L-glutamate, 2-ethylhexyl succinate, diisobutyl adipate, 2-hexyldecyl adipate, di-2-heptylundecyl adipate, diisopropyl sebacate, di-2-ethylhexyl sebacate, ethylene glycol di-2-ethylhexanoate, neopentyl glycol dicaprate, neopentyl glycol dioctanoate, acetoglyceride, glyceryl di-2-heptylundecanoate, glyceryl trioctanoate, glyceryl tri-2-ethylhexanoate, glyceryl trimyristate, glyceryl triisopalmitate, glyceryl tri-2-heptylundecanoate, trimethylolpropane tri-2-ethylhexanoate, trimethylolpropane triisostearate, pentaerythrityl tetraoctanoate, and pentaerythrityl tetra-2-ethylhexanoate; hydrocarbon oils such as liquid paraffin, ozokerite, squalene, pristane, and polybutene; unsaturated fatty acid such as oleic acid, linoleic acid, and linolenic acid; branched fatty acids such as isostearic acid, isopalmitic acid, and isomyristic acid; and silicone oils including linear polysiloxanes such as dimethylpolysiloxane, methylphenylpolysiloxane, and diphenylpolysiloxane, cyclic polysiloxanes such as octamethylcyclotetrasiloxane, decamethylcyclopentasiloxane, and dodecamethylcyclohexasiloxane, and various modified polysiloxanes such as amino-modified polysiloxane, polyether-modified polysiloxane, alkyl-modified polysiloxane, and fluorine-modified polysiloxane. These liquid oil components can be blended either alone or in combination of two or more.

In the present invention, it is preferable that one or more higher fatty acids are blended in (C) the oil component. By blending the higher fatty acid these in the oil component, the emulsion particles become much finer, and a more stable O/W emulsion composition can be obtained. As the higher fatty acid, those having 16 to 24 carbon atoms are preferable. Examples thereof include unsaturated fatty acids such as oleic acid, linoleic acid, and linolenic acid; isostearic acid, isopalmitic acid, isomyristic acid, behenic acid, stearic acid, palmitic acid, and myristic acid. The content of higher fatty acids in the O/W emulsion composition is preferably 0. to 3 mass % and more preferably 0.2 to 1.5 mass %.

The water phase, comprising (D) water, used in the present invention is not limited in particular so far as the main medium of the water phase is water, or water with an aqueous solvent. In addition to water or an aqueous solvent, the components normally used in cosmetics, pharmaceuticals, etc. may be blended thereto within the quantity range that does not affect the stability.

The total blending quantity of water in the O/W emulsion composition of the present invention is not limited in particular. It is generally preferable that the blending quantity is 40 to 95 mass % of the total amount of the O/W emulsion composition.

It is preferable in the production method of the present invention that the water phase comprises an aqueous solvent. The aqueous solvent is not limited in particular and suitably selected from publicly known aqueous solvents depending upon the types of (C) an oil component and (A) a nonionic surfactant. Here, the aqueous solvent means a substance that is liquid at room temperature and water-soluble. Examples thereof include polypropylene glycol polyethylene glycol copolymer or its dimethyl ether, polyethylene glycol or its alkyl ethers, dipropylene glycol, isoprene glycol, and propylene glycol.

In the combination of (C) an oil component and an aqueous solvent used in the present invention, it is necessary that the aqueous solvent is immiscible with the oil component. Examples of such combinations include the followings: when (C) the liquid oil component is dimethylpolysiloxane, the aqueous solvent is polypropylene glycol polyethylene glycol copolymer or its dimethyl ether, polyethylene glycol or its ethyl ether, 1,3-butylene glycol, dipropylene glycol, isoprene glycol, etc.; when (C) is cyclodimethicone (pentamer), the aqueous solvent is polypropylene glycol polyethylene glycol copolymer or its dimethyl ether, polyethylene glycol or its ethyl ether, etc.; when (C) is methylphenylpolysiloxane, the aqueous solvent is polypropylene glycol polyethylene glycol copolymer or its dimethyl ether, 1,3-butylene glycol, glycerin, etc.; when (C) is liquid paraffin, the aqueous solvent is polypropylene glycol polyethylene glycol copolymer or its dimethyl ether, polyethylene glycol or its ethyl ether, 1,3-butylene glycol, dipropylene glycol, isoprene glycol, etc.

As specific examples of the aqueous solvent used in the present invention, the aqueous solvents that have zero to three hydroxyl groups in the molecule can be listed. More specific examples include polypropylene glycol polyethylene glycol copolymer or its alkyl ethers, polyethylene glycol or its alkyl ethers, polyoxyalkylene dicarboxylates, 1,3-butylene glycol, dipropylene glycol, isoprene glycol, 1,2-pentane glycol, 1,2-hexane glycol, 2-methyl-1,3-propanol, ethyl carbitol, 1,2-butylene glycol, glycerin, etc. The aqueous solvent can be suitably selected and used from these solvents depending upon the types of (C) an oil component and (A) a nonionic surfactant. As the aqueous solvent used in the present invention, two or more of these solvents can be used in combination.

On the other hand, the water-soluble materials that have four or more hydroxyl groups in the molecule usually become solid at room temperature, and cannot often achieve the effect of the aqueous solvent in the present invention.

The blending quantity of the aqueous solvent used in the present invention is not limited in particular. Preferably, it is 5 mass % or higher of the emulsified part prepared at 70 °C or higher. If the blending quantity is less than 5 mass %, the preparation of a stable O/W emulsion composition tends to be difficult.

In the production method of the present invention, the main water phase contains (D) water, and it can suitably contain an aqueous solvent, etc. as described above. The blending quantity of the main water phase varies depending on the constitution of the initially prepared water phase (a portion of the water phase), α-gel forming surfactant, etc.; however, it is 50 to 75 mass % of the total amount of the O/W emulsion composition, and preferably 55 to 70 mass %. If the blending quantity of the main water phase is less than 50 mass %, a satisfactory cooling effect cannot be obtained, and the temperature immediately after the completion of the mixing becomes high. Thus, the temperature will not become lower than the α-gel formation temperature range, and the intended O/W emulsion composition cannot be obtained. If the blending quantity exceeds 75 mass %, the water phase in the O/W emulsified part, which is prepared at 70 °C or higher, becomes small. Thus, the preparation of a stable O/W emulsion composition tends to become difficult.

The temperature of the main water phase, which is added afterward, is 10 to 35 °C and most preferably 15 to 30 °C. If the temperature is less than 10 °C, the energy necessary to cool the main water phase becomes excessive, and it tends to be less economical. If the temperature exceeds 35 °C, the temperature immediately after the completion of the mixing of the main water phase becomes high, the temperature will not become the lower temperature limit, or lower, of the α-gel formation temperature range; thus the intended O/W emulsion composition cannot be obtained.

The α-gel formation temperature range in the production method of the present invention depends on the types of blended surfactant and higher alcohol, the constitution of the water phase, etc.; however, it is typically about 37 to 52 °C. When the emulsified part is prepared with the use of the oil phase that does not contain a liquid oil component, the α-gel formation temperature range is typically about 44 to 60 °C.

It can be confirmed by DSC (differential scanning calorimetry) that the oil phase forms an α-gel in the water phase. The emulsified part obtained by emulsifying the oil phase comprising (A) a hydrophilic nonionic surfactant, (B) a linear higher alcohol having 16 or more carbon atoms, and (C) an oil component in the water phase is measured by DSC (differential scanning calorimetry) while lowering the temperature to 70 °C or lower. Then an exothermic peak is observed, and this indicates that the oil phase has formed an α-gel in the water phase.

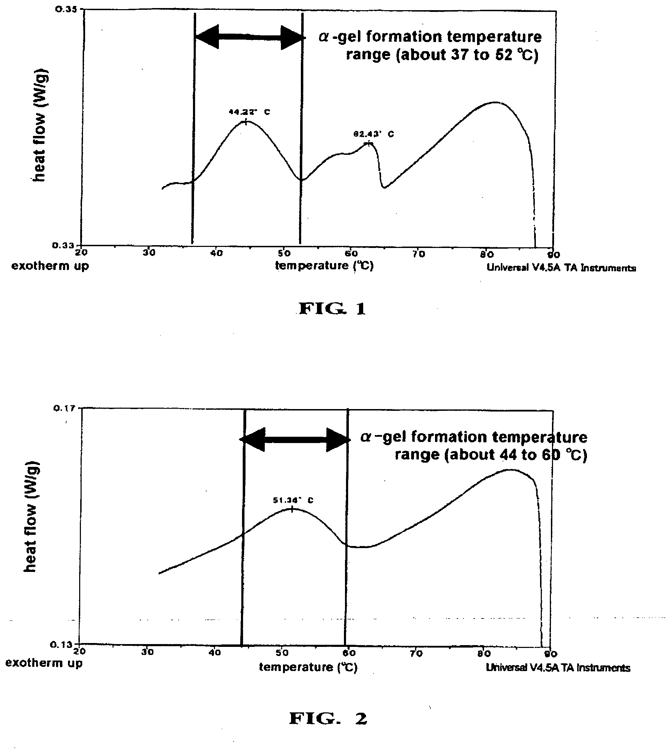

"The α-gel formation temperature range" in the present invention means the temperature range of the exothermic peak due to the formation of α-gel. The peak temperature means the temperature at the top of the exothermic peak (refer to Figs. 1 and 2). In the DSC measurement, other exothermic peaks may be detected in addition to the exothermic peak due to the formation of α-gel. These are exothermic peaks due to simple solidification of components contained in the emulsified part. The exothermic peak due to the formation of α-gel is different from these exothermic peaks due to solidification.

The peak temperature of the exothermic peak measured by DSC (differential scanning calorimetry) is considered to correspond to the melting point of the emulsified part in the production method of the present invention. The lower temperature limit of the α-gel formation temperature range in the production method of the present invention is normally located at a point that is 2 to 4 °C lower than the peak temperature. When the oil phase from which at least a portion of liquid oil components is excluded is used for the preparation of the emulsified part, the lower temperature limit of the α-gel formation temperature range is normally located at a point that is 6 to 8 °C lower than the peak temperature of the emulsified part.

Accordingly, as the lower temperature limit of the α-gel formation temperature range, the temperature that is 4 °C lower than the exothermic peak temperature of the α-gel formation (when the emulsified part is prepared with the use of the oil phase comprising a liquid oil component) is adopted, or the temperature that is 8 °C lower (when the emulsified part is prepared with the use of the oil phase from which at least a portion of the liquid oil component is excluded) can be adopted. When the temperature becomes lower than this, the stirring may be stopped and it can be left to cool. In the case where the liquid oil component is added afterward, the liquid oil component is added after the temperature becomes the lower temperature limit or lower of the α-gel formation temperature range, and uniformly mixed. Then, the stirring may be stopped and it can be left to cool.

The O/W emulsion composition prepared by the production method of the present invention is in a state that an associated complex consisting of lamellar bimolecular membranes formed from a nonionic surfactant and a higher alcohol, namely α-gel, is present in the interface of emulsion particles formed from a nonionic surfactant, a higher alcohol, and an oil component in the presence of water.

Hereinafter, the concept for the production method of the O/W emulsion composition of the present invention will be explained.

### Concept:

(1) An O/W emulsion composition can be obtained by the process in that an oil phase comprising (A) a nonionic surfactant, (B) a linear higher alcohol that has 16 or more carbon atoms and can form an α-gel in water with the nonionic surfactant, and (C) an oil component is emulsified, at 70 °C or higher, with a water phase comprising (D) water, and then the obtained emulsion is cooled with continuous stirring until the temperature becomes lower than the temperature range wherein the oil phase forms an α-gel in the water phase.
(2) However, if the emulsion is left to cool, a considerable amount of time is necessary. If the cooling takes too much time in the α-gel formation temperature range, the emulsion particles aggregate to provide a highly viscous emulsion composition, and the intended O/W emulsion composition may not be obtained. It is also economically disadvantageous if a cooling apparatus such as an Onlator is used. Accordingly, in order to solve the above-described problem, a highly concentrated emulsifed part is prepared at 70 °C or higher (preferably 70 to 80 °C, and more preferably 70 to 75 °C) with the use of a portion of the water phase. The remaining water phase (main water phase) at 10 to 35 °C is gradually mixed to the emulsified part, with stirring, until the temperature becomes lower than the lower temperature limit of the α-gel formation temperature range. By the mixing of this low-temperature main water phase, the high-temperature emulsified part is rapidly cooled as well as diluted. When the stirring is continued until the temperature becomes the lower temperature limit, or lower, of the α-gel formation temperature range, an O/W emulsion composition wherein the α-gel is present in the interface of emulsion particles is prepared. When the lower temperature limit, or lower, of the α-gel formation temperature range is reached, the stirring can be stopped and the emulsion composition can be left to cool. The apparatuses used in emulsification and stirring can suitably be selected from normally used apparatuses. The thus far used apparatuses can be applied, and no special equipment is necessary.
(3) Depending upon the formulation of the intended O/W emulsion composition, there may be a case where the temperature of the mixture obtained by the mixing with stirring of the main water phase at 10 to 35 °C becomes the lower temperature limit, or lower, of the α-gel formation temperature to cause the aggregation of the emulsion particles, and the intended O/W emulsion composition can not be obtained. In this case, a highly concentrated emulsified part is prepared with the use of the oil phase from which at least a portion of liquid oil components is excluded, and the remaining liquid oil components can be added after cooling in the same way as above.

This is because the α-gel formation temperature range shifts to the higher-temperature side by excluding the liquid oil components from the oil phase components of the emulsified part. Such an emulsified part that has the α-gel formation temperature range shifted to the higher-temperature side can be easy to be adjusted to the α-gel formation temperature range or lower when the main water phase is mixed with stirring. In order to obtain such an effect, it is preferable that no liquid oil component is contained in the oil phase used for the preparation of the emulsified part, or the liquid oil component contained in the oil phase used for the preparation of the emulsified part is less than 5 mass % of the total amount of the liquid component.

The O/W emulsion composition obtained by the above-described production method can stably exist over a wide temperature range for a long period of time though the particle size is small, ranging from 1 to 7.5 µm.

In the conventional production method of an O/W emulsion composition, the following method has been used: water, a moisturizer, and a thickener are dissolved and heated to about 70 °C to prepare a water phase in advance; an oil phase obtained by uniform mixing an oil component, a higher alcohol, and a hydrophilic nonionic surfactant, at about 70 °C, is emulsified into the water phase while being stirred with a homogenizer; and the obtained emulsion is rapidly cooled with an Onlator to about 35 °C. However, the energy use in the conventional production method is wasteful because of heating and the use of a heat exchanger, and the consumption of water used for the cooling machine is large; thus the environmental burden has been high.

On the other hand, the O/W emulsion composition obtained by the production method of the present invention can be easily produced with low energy because a cooling machine such as an Onlator is not used when emulsification, and it is not necessary to heat a large amount of water or an aqueous solvent. In addition, the emulsification is virtually carried out only with a nonionic surfactant, whose irritation to the human body is relatively small; thus it has excellent safety.

As mentioned above, in the production method of the O/W emulsion composition of the present invention, the preparation temperature can be controlled only by adding the water phase at 10 to 35 °C to the emulsified part produced in advance at 70 to 80 °C, and an excellent O/W emulsion composition with the same usability as the conventional one can be obtained. Thus, the conventional production process can be drastically simplified.

The O/W emulsion composition of the present invention can be preferably applied to external preparations used on the body such as the skin and hair in the fields such as cosmetics, pharmaceuticals, and quasi-drugs. For example, it can be used for products such as skin cosmetics, hair cleanser, skin cleanser and hair dressing.

In the O/W emulsion composition of the present invention, in addition to the above-described essential components, the components normally used in cosmetics, pharmaceuticals, etc. can be blended within the range that the effect of the present invention, such as stability, is not undermined. Examples of such components include the following:

Moisturizers such as polyethylene glycol and its alkyl ethers, glycerin, sorbitol, xylitol, maltitol, mucopolysaccharides, hyaluronic acid, chondroitin sulfate, and chitosan. Thickeners such as methylcellulose, ethylcellulose, gum arabic, and polyvinyl alcohol. Organic solvents such as ethanol. Antioxidants such as butylhydroxytoluene, tocopherol, and phytic acid. Antimicrobial preservative agents such as benzoic acid, salicylic acid, sorbic acid, paraoxybenzoic acid ester (ethylparaben, butylparaben, etc.), and hexachlorophene. Amino acids such as glycine, alanine, valine, leucine, serine, threonine, phenylalanine, tyrosine, aspartic acid, asparagine, glutamine, taurine, arginine, and histidine; and their salts. Organic acids such as acyl sarcosinic acid (for example, sodium lauroyl sarcosinate), glutathione, citric acid, malic acid, tartaric acid, and lactic acid.

Vitamin A and its derivatives; vitamin Bs such as vitamin B₆ hydrochloride, vitamin B₆ tripalmitate, vitamin B₆ dioctanoate, vitamin B₂ and its derivatives, vitamin B₁₂, and vitamin B₁₅, and its derivatives; vitamin Cs such as ascorbic acid, ascorbyl phosphate ester (its salts), and ascorbyl dipalmitate; vitamin Es such as α-tocopherol, β-tocopherol, γ-tocopherol, vitamin E acetate, and vitamin E nicotinate; vitamin Ds; and vitamins such as vitamin H, pantothenic acid, and pantethine. Various agents such as nicotinic acid amide, benzyl nicotinate, γ-oryzanol, allantoin, glycyrrhizic acid (its salts), glycyrrhetinic acid and its derivatives, hinokitiol, mucidin, bisabolol, eucalyptol, thymol, inositol, saponins (saikosaponin, ginseng saponin, luffa cylindrica saponin, sapindus mukorossi saponin, etc.), pantothenyl ethyl ether, ethinylestradiol, tranexamic acid, cepharanthine, and placenta extract.

Natural extracts obtained by the extraction with a solvent such as organic solvents, alcohols, polyhydric alcohols, water, aqueous alcohols, from materials such as sorrel, Sophora flavescens Aiton, cow lily, orange, sage, thyme, yarrow, mallow, cnidium root, Swertia japonica, Angelica acutiloba, spruce, birch, field horsetail, Luffa cylindrica, horse chestnut, saxifrage, arnica, lily, mugwort, peony, aloe, gardenia, and sawara cypress. Cationic surfactants such as stearyltrimethylammanium chloride, benzalkonium chloride, and laurylamine oxide. Sequestering agents such as disodium edetate, trisodium edetate, sodium citrate, sodium polyphosphate, sodium metaphosphate, and gluconic acid. Neutralizing agents such as potassium hydroxide, sodium hydroxide, and triethanolamine.

In addition, Perfumes, scrubbing agents, powder, coloring agents, whitening agents, UV protection agents such as UV absorbers and UV scattering agents, etc. may be appropriately used so far as effects such as stability is not impaired.

### EXAMPLES

Hereinafter, the present invention will be explained in further detail with reference to the examples of O/W emulsion compositions of the present invention. However, the present invention is not limited by these examples. Unless otherwise noted, the blending quantities are all expressed in mass %.

Initially, the methods for the test and the evaluation for each production example will be explained.

### (Viscosity)

With the use of a B-type viscometer (rotor: No. 3, rotation speed: 12 rpm), the viscosity at 30 °C was measured.

### (Stability)

The prepared O/W emulsion composition was stored in a constant temperature bath, at a low temperature (-10 °C) to a high temperature (60 °C), for 1 month. Then, the emulsion state was observed with the naked eye to check for the destruction or coalescence of emulsion particles and the judgment was carried out as follows.
○: The destruction and coalescence of emulsion particles are not observed.
Δ: The destruction or coalescence of emulsion particles is partially observed.
x: The destruction or coalescence of emulsion particles is observed.

Here, Δ or higher was evaluated to be acceptable.

### (Comparability to conventional product)

The usability (spreadability, fit feeling, stickiness, refreshing feeling, and moist feeling) was evaluated by 10 panelists. Based on the number of panelists who answered that the product was comprehensively comparable to the conventional process product (i.e., Production Example 1-13 in Table 1 and Production Example 2-15 in Table 2), the judgment was carried out as follows.
○ : 9 panelists or more and 10 panelists or less
○Δ: 7 panelists or more and 8 panelists or less
Δ : 5 panelists or more and 6 panelists or less
Δx: 3 panelists or more and 4 panelists or less
x : 2 panelists or less

Here, Δ or higher was evaluated to be acceptable.

In the table below, the evaluation results of viscosity, stability, and the comparability to the conventional product, for the O/W emulsion compositions produced by various production methods, and the blending formulation thereof are summarized. The quantities in the table below are expressed in mass %.

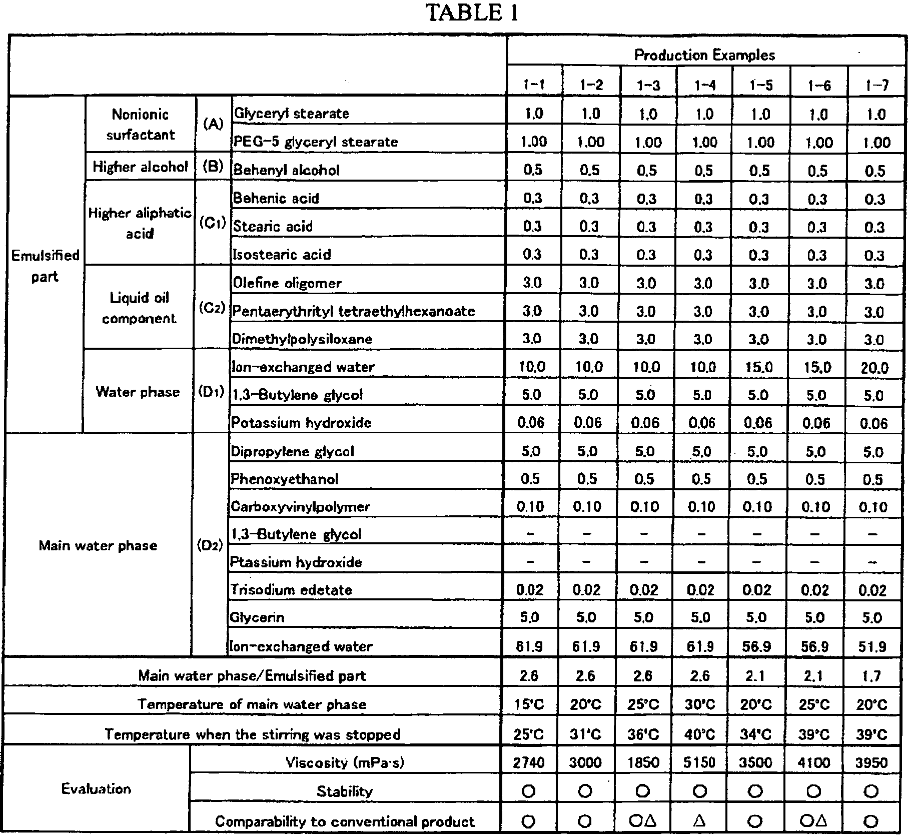

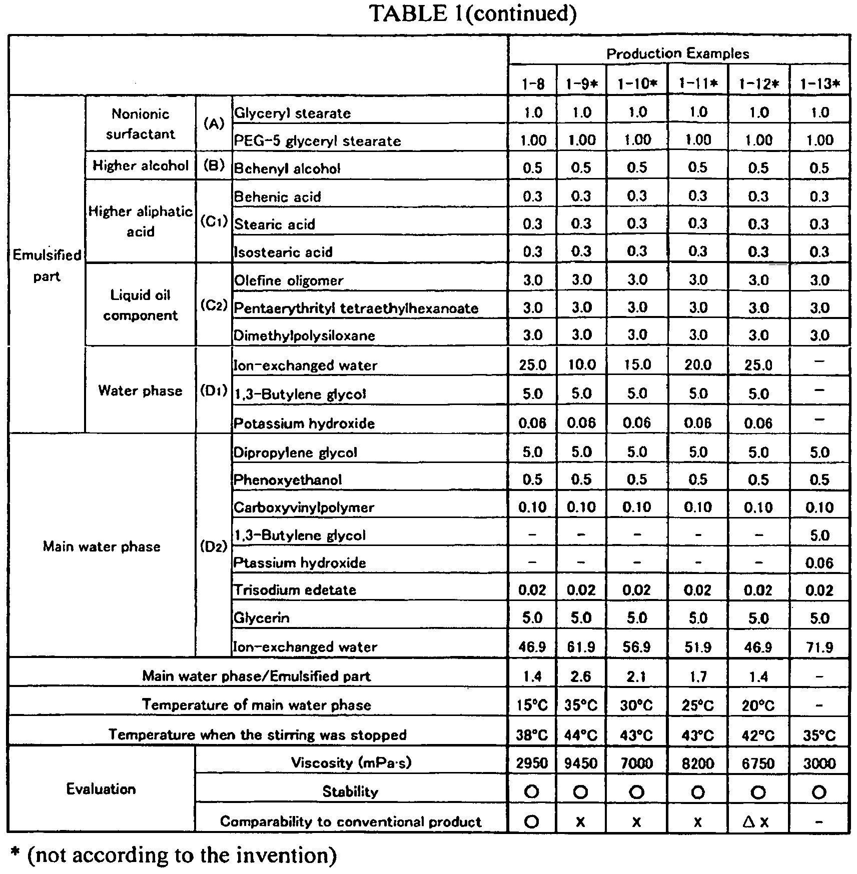

### Production Examples 1-1 to 1-12 (1-9 to 1-12 not according to the invention)

An O/W emulsion (emulsified part) was obtained by emulsifying (A), (B), (C₁), (C₂), and (D₁) listed in the table at 70 °C. While this emulsified part was being stirred, the main water phase (D₂) at a specified temperature was mixed with the emulsified part. The temperature of the mixture at the completion of the mixing of the main water phase was 40.2 °C or lower in all cases. When the specified temperature was reached, the stirring was stopped. Subsequently, the mixture was left to cool to room temperature to obtain an O/W emulsion composition.

### Production Example 1-13 (not according to the invention)

While (A), (B), (C₁), and (C₂) listed in the table were being stirred with a homogenizer, (D₁) and (D₂) were added to the mixture to carried out emulsification at 70 °C. The obtained O/W emulsion was cooled to 35 °C by passing it through an Onlator. Subsequently, it was left to cool to room temperature to obtain an O/W emulsion composition.

The results for the measurement of exothermic peaks of the emulsified part are shown in Fig. 1. The measurement was carried out with a DSC (Q-1000, TA Instruments, USA) by decreasing the temperature at a rate of 2 °C per minute from 70 °C to 30 °C. As a result, the peak temperature of the exothermic peak due to α-gel formation, for the emulsified part having the formulation of the above table, was 44.2 °C as shown in Fig. 1.

As shown in the above Table 1, it was clarified that an O/W emulsion composition nearly comparable to the conventional product (Production Example 1-13) could be obtained in Production Examples 1-1 to 1-8 because the stirring was stopped at the temperature 40.2 °C, or lower, which is 4 °C lower than the exothermic peak temperature (44.2 °C). On the other hand, in Production Examples 1-9 to 1-12, an O/W emulsion composition comparable to the conventional product could not be obtained because the stirring was stopped at a higher temperature than 40.2 °C. Accordingly, in the present invention, the temperature that is 4 °C lower than the exothermic peak temperature of α-gel formation can be adopted as the lower temperature limit of the α-gel formation temperature range. If the temperature is lower than this, an O/W emulsion composition comparable to the conventional product can be obtained even without stirring.

It was also clarified that even when the temperature of the main water phase is relatively high, an O/W emulsion composition comparable to the conventional process product can be obtained if the amount of the main water phase with respect to the emulsified part is large.

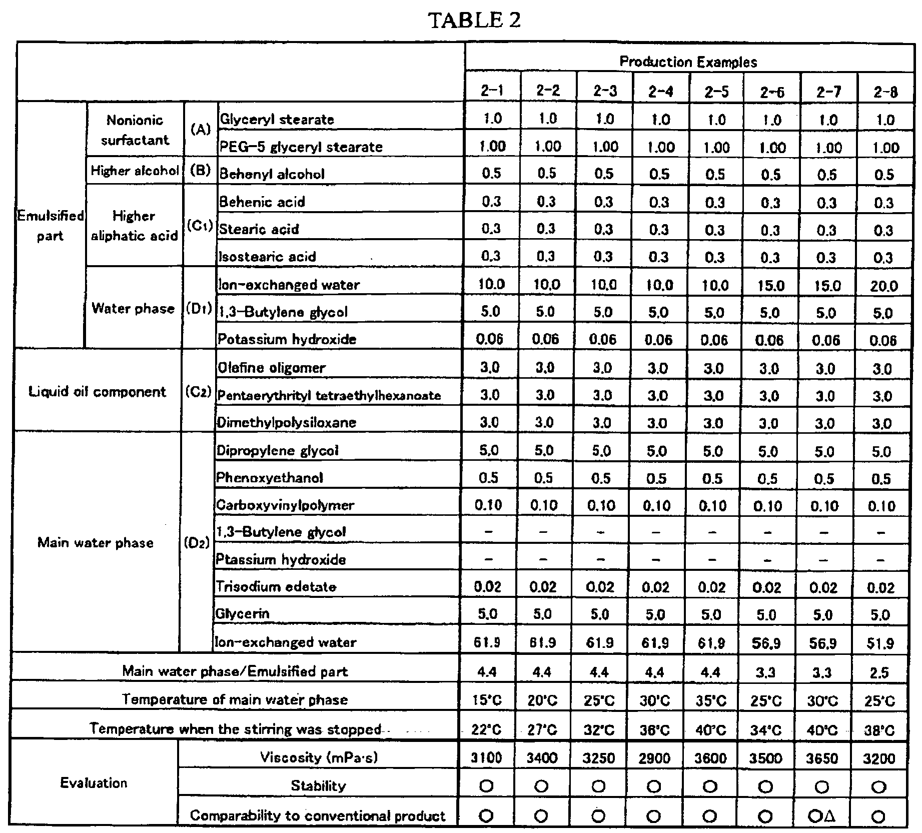

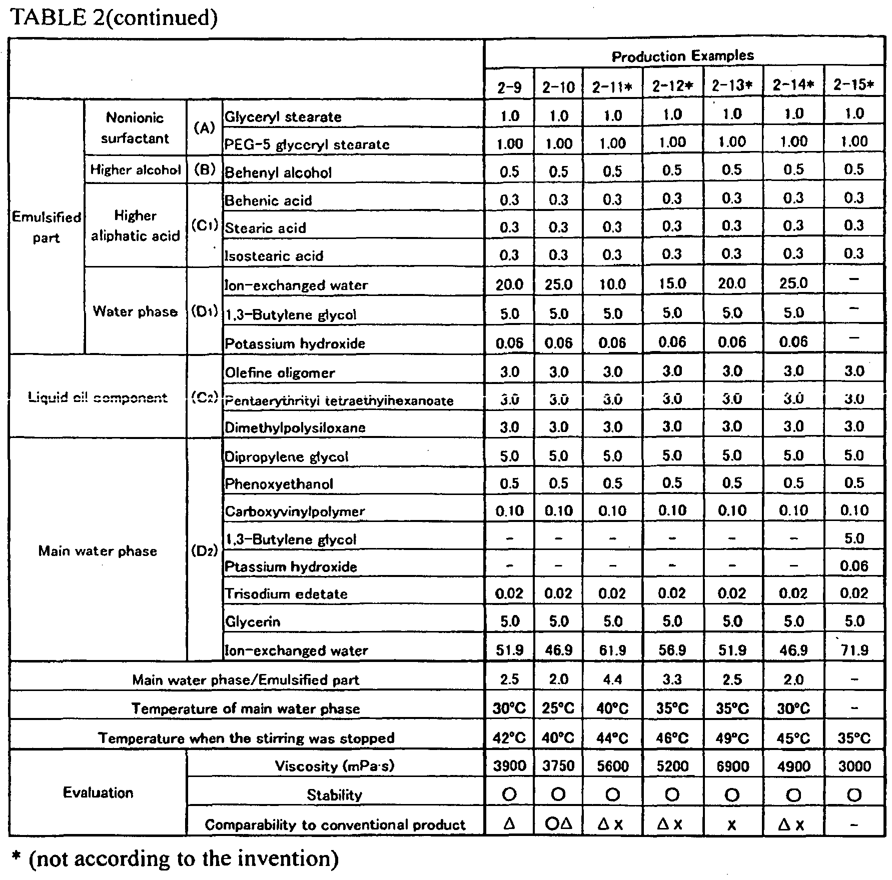

### Production Examples 2-1 to 2-14 (2-11 to 2-14 not according to the invention)

An O/W emulsion (emulsified part) was obtained by emulsifying (A), (B), (C₁), and (D₁) listed in the table at 70 °C. While this emulsified part was being stirred, the main water phase (D₂) at a specified temperature was mixed into the emulsified part. The temperature of the mixture at the completion of mixing was 43.3 °C or lower in all cases. Then, the liquid oil components were added thereto. When the specified temperature was reached, the stirring was stopped. Subsequently, the mixture was left to cool to room temperature to obtain an O/W emulsion composition.

### Production Example 2-15 (not according to the invention)

While (A), (B), (C₁), and (C₂) listed in the table were being stirred with a homogenizer, (D₁) and (D₂) were added to the mixture to carry out emulsification at 70 °C. The obtained O/W emulsion was cooled to 35 °C by passing it through an Onlator. Subsequently, it was left to cool to room temperature to obtain an O/W emulsion composition.

The results for the measurement of exothermic peaks of the emulsified part are shown in Fig. 2. The measurement was carried out with a DSC (Q-1000, TA Instruments, USA) by decreasing the temperature at a rate of 2 °C per minute from 70 °C to 30 °C. As a result, the exothermic peak for the emulsified part having the formulation of the above table was at 51.3 °C. Thus, it was found that, because the oil phase from which at least a portion of liquid oil components was excluded was used, the α-gel formation temperature range of the emulsified part shifted to the higher-temperature side overall, compared with the case in which the oil phase that contains the entire liquid oil components was used, and the exothermic peak temperature shifted to the higher-temperature side (about 7 °C).

As shown in the above Table 2, it was clarified that an O/W emulsion composition nearly comparable to the conventional product (Production Example 2-15) could be obtained in Production Examples 2-1 to 2-10 because the stirring was stopped at the temperature 43.3 °C, or lower, which is 8 °C lower than the exothermic peak (51.3 °C). Accordingly, it was clarified that when the emulsified part was prepared by using the oil phase from which at least a portion of liquid oil components was excluded, the temperature that is 8 °C lower than the exothermic peak temperature of α-gel formation could be adopted as the lower temperature limit of the α-gel formation temperature range, and the stirring could be stopped at an earlier stage compared with the case in that the emulsified part is prepared from the oil phase containing the liquid oil components.

On the other hand, in Production Examples 2-11 to 2-14, an O/W emulsion composition comparable to the conventional product could not be obtained because the stirring was stopped at a higher temperature than 43.3 °C.

It was also clarified that even when the temperature of the main water phase is relatively high, an O/W emulsion composition comparable to the conventional process product can be obtained if the amount of the main water phase with respect to the emulsified part is large.

Thus, it was found that according to the production method of the present invention, an O/W emulsion composition could easily be produced with low energy without using a cooling machine such as an Onlator: it was very economical. In addition, the O/W emulsion composition produced by the production method of the present invention has comparable quality and the usability to the O/W emulsion composition that has the same formulation and is prepared with the use of a cooling machine such as an Onlator.

Hereinafter, the present invention will be further explained by examples. However, the present invention is not limited by these examples. The quantities in the following formulations are expressed in mass %.

### Example 1

| Milky lotion | Blending quantity (mass %) |
|---|---|
| (1) Ion-exchanged water | balance |
| (2) Glycerin | 5.0 |
| (3) Butylene glycol | 5.0 |
| (4) Polyethylene glycol 1500 | 2.0 |
| (5) Ethanol | 3.0 |
| (6) Phenoxyethanol | 0.3 |
| (7) Paraben | 0.1 |
| (8) Potassium hydroxide | 0.1 |
| (9) Trisodium edetate | 0.05 |
| (10) Carboxyvinyl polymer | 0.1 |
| (11) Xanthan gum | 0.1 |
| (12) Behenyl alcohol | 0.5 |
| (13) Behenic acid | 0.3 |
| (14) Stearic acid | 0.4 |
| (15) Isostearic acid | 0.3 |
| (16) Petrolatum | 2.0 |
| (17) Squalane | 3.0 |
| (18) Decamethylcyclopentasiloxane | 3.0 |
| (19) Dimethylpolysiloxane | 2.0 |
| (20) Cetyl 2-ethylhexanoate | 2.0 |
| (21) PEG-60 glyceryl isostearate | 1.0 |
| (22) PEG-5 glyceryl stearate | 1.0 |
| (23) Perfume | 0.1 |

### Production method:

The water phase (a portion of water phase) consisting of a portion of (1) to (3) (i.e., about 25 mass % of the total amount of (1) to (3)) and (8) was heated to 70 °C. The oil phase consisting of (12) to (16), (21), and (22) was heated to 70 °C. The oil phase was gradually added to the water phase and emulsified with a homomixer to prepare an emulsified part. While the emulsified part was being stirred, the remaining water phase components (main water phase) of (1) to (11) at 25 °C, and the liquid oil components consisting of (17) to (20) and (23) were added and mixed, in order, into the emulsified part to obtain a milky lotion. The viscosity of the milky lotion was 3,000 mPa·s/30 °C. (peak temperature in the α-gel formation temperature range: 52 °C, temperature when the stirring for mixing was stopped: 38 °C)

### Example 2

| Milky Lotion | Blending quantity (mass%) |
|---|---|
| (1)Ion-exchanged water | balance |
| (2)Glycerin | 5.0 |
| (3)Butylene glycol | 5.0 |
| (4)Polyethylene glycol 1500 | 2.0 |
| (5)Ethanol | 3.0 |
| (6)Phenoxyethanol | 0.3 |
| (7)Paraben | 0.1 |
| (8)Potassium hydroxide | 0.1 |
| (9)Trisodium edetate | 0.05 |
| (10)Carboxyvinyl polymer | 0.1 |
| (11)Xanthan gum | 0.1 |
| (12)Behenyl alcohol | 0.5 |
| (13)Behenic acid | 0.3 |
| (14)Stearic acid | 0.4 |
| (15)Isostearic acid | 0.3 |
| (16)Petrolatum | 2.0 |
| (17)Squalane | 3.0 |
| (18)Decamethylcyclopentasiloxane | 3.0 |
| (19)Dimethylpolysiloxane | 2.0 |
| (20)Cetyl 2-ethylhexanoate | 2.0 |
| (21)per-60 glyceryl isostearate | 1.0 |
| (22)PEG-5 glyceryl stearate | 1.0 |
| (23)Perfume | 0.1 |

### Production method:

The water phase (a portion of water phase) consisting of a portion of (1) to (3) (i.e., about 25 mass % of the total amount of (1) to (3)) and (8) was heated to 70 °C. The oil phase consisting of (12) to (23) was heated to 70 °C. The oil phase was gradually added to the water phase and emulsified with a homomixer to prepare an emulsified part. While the emulsified part was being stirred, the remaining water phase components (main water phase) of (1) to (7) and (9) to (11) at 25 °C were added and mixed into the emulsified part to obtain a milky lotion. The viscosity of the milky lotion was 3,000 mPa·s/30 °C. (peak temperature in the α-gel formation temperature range: 44 °C, temperature when the stirring for mixing was stopped: 39 °C)

### Example 3

| Cream | Blending quantity (mass%) |
|---|---|
| (1)Ion-exchanged water | balance |
| (2)Glycerin | 7.0 |
| (3)Dipropylene glycol | 7.0 |
| (4)Erythritol | 1.0 |
| (5)Polyethylene glycol 20000 | 2.0 |
| (6)Phenoxyethanol | 0.5 |
| (7)Triethanolamine | 0.5 |
| (8)Trisodium edetate | 0.1 |
| (9)Carboxyvinyl polymer | 0.1 |
| (10)Xanthan gum | 0.1 |
| (11)Behenyl alcohol | 3.0 |
| (12)Stearyl alcohol | 1.0 |
| (13)Behenic acid | 0.2 |
| (14)Stearic acid | 0.5 |
| (15)Isostearic acid | 0.3 |
| (16)Microcrystalline wax | 1.0 |
| (17)Petrolatum | 2.0 |
| (18)Squalane | 5.0 |
| (19)Decamethylcyclopentasiloxane | 3.0 |
| (20)Dimethylpolysiloxane | 3.0 |
| (21)Isononyl isononanoate | 2.0 |
| (22)PEG-60 glyceryl isostearate | 1.0 |
| (23)PEG-5 glyceryl stearate | 1.0 |
| (24)Perfume | 0.1 |

### Production method:

The water phase (a portion of water phase) consisting of a portion of (1) to (3) (i.e., about 30 mass % of the total amount of (1) to (3)) and (7) was heated to 70 °C. The oil phase consisting of (11) to (17), (22), and (23) was heated to 70 °C. The oil phase was gradually added to the water phase and emulsified with a homomixer to prepare an emulsified part. While the emulsified part was being stirred, the remaining water phase components (main water phase) of (1) to (10) at 25 °C, and the liquid oil components consisting of (18) to (21) and (24) were added and mixed, in order, into the emulsified part to obtain a cream. The viscosity of the cream was 30,000 mPa·s/30 °C. (peak temperature in the α-gel formation temperature range: 66 °C, temperature when the stirring for mixing was stopped: 42 °C)

### Example 4

| Cream | Blending quantity (mass%) |
|---|---|
| (1)Ion-exchanged water | balance |
| (2)Glycerin | 7.0 |
| (3)Dipropylene glycol | 7.0 |
| (4)Erythritol | 1.0 |
| (5)Polyethylene glycol 20000 | 2.0 |
| (6)Phenoxyethanol | 0.5 |
| (7)Triethanolamine | 0.5 |
| (8)Trisodium edetate | 0.1 |
| (9)Carboxyvinyl polymer | 0.1 |
| (10)Xanthan gum | 0.1 |
| (11)Behenyl alcohol | 3.0 |
| (12)Stearyl alcohol | 1.0 |
| (13)Behenic acid | 0.2 |
| (14)Stearic acid | 0.5 |
| (15)Isostearic acid | 0.3 |
| (16)Microcrystalline wax | 1.0 |
| (17)Petrolatum | 2.0 |
| (18)Squalane | 5.0 |
| (19)Decamethylcyclopentasiloxane | 3.0 |
| (20)Dimethylpolysiloxane | 3.0 |
| (21)Isononyl isononanoate | 2.0 |
| (22)PEG-60 glyceryl isostearate | 1.0 |
| (23)PEG-5 glyceryl stearate | 1.0 |
| (24)Perfume | 0.1 |

### Production method

The water phase (a portion of water phase) consisting of a portion of (1) to (3) (i.e., about 15 mass % of the total amount of (1) to (3)) and (7) was heated to 70 °C. The oil phase consisting of (11) to (24) was heated to 70 °C. The oil phase was gradually added to the water phase and emulsified with a homomixer to prepare an emulsified part. While the emulsified part was being stirred, the remaining water phase components (main water phase) of (1) to (6) and (8) to (10) at 25 °C were added and mixed into the emulsified part to obtain a cream. The viscosity of the cream was 30,000 mPa·s/30 °C. (peak temperature in the α-gel formation temperature range: 61 °C, temperature when the stirring for mixing was stopped: 44 °C)

### Example 5

| Sunscreen | Blending quantity (mass%) |
|---|---|
| (1)Polyoxylethylene hydrogenated castor oil | 1.0 |
| (2)Dimethicone copolyol | 0.5 |
| (3)Decamethylcyclopentasiloxane | 15.0 |
| (4)Behenic acid | 0.3 |
| (5)Stearic acid | 0.2 |
| (6)Behenyl alcohol | 0.3 |
| (7)Phenyltrim ethi cone | 1.0 |
| (8)Hydrophobized titanium oxide | 5.0 |
| (9)Hydrophobized zinc oxide | 2.0 |
| (10)spherical alkyl polyacrylate powder | 2.0 |
| (11)2-Ethylhexyl paramethoxycinnamate | 5.0 |
| (12)Citric acid | 0.01 |
| (13)Sodium citrate | 0.09 |
| (14)Silica | 1.0 |
| (15)Paraben | 0.1 |
| (16)Phenoxyethanol | 0.3 |
| (17)Sodium hydroxide | 0.05 |
| (18)Alcohol | 5.0 |
| (19)Dynamite glycerin | 1.0 |
| (20)Succinoglucan | 0.2 |
| (21)Cellulose gum | 1.0 |
| (22)Ion-exchanged water | balance |

### Production method:

The water phase (a portion of water phase) consisting of a portion of (19) and (22) (i.e., about 20 mass % of the total amount of (19) and (22)), (1) and (17) was heated to 70 °C. The oil phase consisting of (2) and (4) to (6) was heated to 70 °C. The oil phase was gradually added to the water phase and emulsified with a homomixer to prepare an emulsified part. While the emulsified part was being stirred, the remaining water phase components (main water phase) of (12) to (22) at 25 °C, and the liquid oil components and powder components of (2) and (7) to (11) were added and mixed, in order, into the emulsified part to obtain a sunscreen. The viscosity of the sunscreen was 5,000 mPa·s/30 °C. (peak temperature in the α-gel formation temperature range: 51 °C, temperature when the stirring for mixing was stopped: 38 °C)

### Example 6

| Sunscreen | Blending quantity (mass%) |
|---|---|
| (1)Polyoxylethylene hydrogenated castor oil | 1.0 |
| (2)Dimethicone copolyol | 0.5 |
| (3)Decamethylcyclopentasiloxane | 15.0 |
| (4)Behenic acid | 0.3 |
| (5)Stearic acid | 0.2 |
| (6)Behenyl alcohol | 0.3 |
| (7)Phenyltrimethicone | 1.0 |
| (8)Hydrophobized titanium oxide | 5.0 |
| (9)Hydrophobized zinc oxide | 2.0 |
| (10)spherical alkyl polyacrylate powder | 2.0 |
| (11)2-Ethylhexyl paramethoxycinnamate | 5.0 |
| (12)Citric acid | 0.01 |
| (13)Sodium citrate | 0.09 |
| (14)Silica | 1.0 |
| (15)Paraben | 0.1 |
| (16)Phenoxyethanol | 0.3 |
| (17)Sodium hydroxide | 0.05 |
| (18)Alcohol | 5.0 |
| (19)Dynamite glycerin | 1.0 |
| (20)Succinoglucan | 0.2 |
| (21)Cellulose gum | 1.0 |
| (22)Ion-exchanged water | balance |

### Production method:

The water phase (a portion of water phase) consisting of a portion of (19) and (22) (i.e., about 20 mass % of the total amount of (19) and (22)) and (17) was heated to 70 °C. The oil phase consisting of (1) to (7) and (11) was heated to 70 °C. The oil phase was gradually added to the water phase and emulsified with a homomixer to prepare an emulsified part. While the emulsified part was being stirred, the remaining water phase components (main water phase) at 25 °C were added and mixed into the emulsified part to obtain a sunscreen. The viscosity of the sunscreen was 5,000 mPa·s/30 °C. (peak temperature in the α-gel formation temperature range: 45 °C, temperature when the stirring for mixing was stopped: 39 °C)

### Example 7

| Emulsion foundation | Blending quantity (mass%) |
|---|---|
| (1)Talc | 3.0 |
| (2)Titanium dioxide | 4.0 |
| (3)Red iron oxide | 0.5 |
| (4)Yellow iron oxide | 1.5 |
| (5)Black iron oxide | 0.1 |
| (6)Bentonite | 0.5 |
| (7)Polyoxyethylene sorbitan monostearate | 1.0 |
| (8)Triethanolamine | 1.5 |
| (9)Dipropylene glycol | 8.0 |
| (10)Ion-exchanged water | balance |
| (11)Behenic acid | 0.5 |
| (12)Stearyl alcohol | 0.4 |
| (13)Isohexadecyl alcohol | 6.0 |
| (14)Glyceryl monostearate | 2.0 |
| (15)Liquid lanolin | 2.0 |
| (16)Liquid paraffin | 6.0 |
| (17)Paraben | 0.1 |
| (18)Perfume | 0.05 |

### Production method:

The mixture of (8) and (9) in which (6) and (17) were dispersed was added to a portion of (10) and stirred at 70 °C. The oil phase consisting of (7), (11), (12) and (14) was dissolved with heating at 70 °C. The oil phase was gradually added to the mixture and emulsified with a homomixer to prepare an emulsified part. While the emulsified part was being stirred, the remaining (10) at room temperature, the liquid oil components of (13),(15), (16) and (18), and sufficiently mixed and pulverized powder components of (1) to (5) were added and mixed into the emulsified part to obtain an emulsion foundation. The viscosity of the emulsion foundation was 5,000 mPa·s/30 °C. (peak temperature in the α-gel formation temperature range: 51 °C, temperature when the stirring for mixing was stopped: 41 °C)

### Example 8

| Emulsion foundation | Blending quantity (mass%) |
|---|---|
| (1)Talc | 3.0 |
| (2)Titanium dioxide | 4.0 |
| (3)Red iron oxide | 0.5 |
| (4)Yellow iron oxide | 1.5 |
| (5)Black iron oxide | 0.1 |
| (6)Bentonite | 0.5 |
| (7)Polyoxyethylene sorbitan monostearate | 1.0 |
| (8)Triethanolamine | 1.5 |
| (9)Dipropylene glycol | 8.0 |
| (10)Ion-exchanged water | balance |
| (11)Behenic acid | 0.5 |
| (12)Stearyl alcohol | 0.4 |
| (13)Isohexadecyl alcohol | 6.0 |
| (14)Glyceryl monostearate | 2.0 |
| (15)Liquid lanolin | 2.0 |
| (16)Liquid paraffin | 6.0 |
| (17)Paraben | 0.1 |
| (18)Perfume | 0.05 |

### Production method:

The mixture of (8) and (9) in which (6) and (17) were dispersed was added to a portion of (10) and stirred at 70 °C. The oil phase consisting of (7), (11) to (16) and (18) was dissolved with heating at 70 °C. The oil phase was gradually added to the mixture and emulsified with a homomixer to prepare an emulsified part. While the emulsified part was being stirred, the remaining (10) at room temperature and sufficiently mixed and pulverized powder components of (1) to (5) were added and mixed into the emulsified part to obtain an emulsion foundation. The viscosity of the emulsion foundation was 5,000 mPa·s/30 °C. (peak temperature in the α-gel formation temperature range: 46 °C, temperature when the stirring for mixing was stopped: 42 °C)

### Example 9

| Emulsion eyeshadow | Blending quantity (mass%) |
|---|---|
| (1)Talc | 10.0 |
| (2)Kaolin | 2.0 |
| (3)Pigment | 5.0 |
| (4)Stearic acid | 1.0 |
| (5)Behenyl alcohol | 0.2 |
| (6)Isopropyl myristate | 6.0 |
| (7)Liquid paraffin | 5.0 |
| (8)Propyleneglycol monolaurate | 3.0 |
| (9)Perfume | 0.05 |
| (10) Ion-exchanged water | balance |
| (11)Butylene glycol | 5.0 |
| (12)Glycerin | 1.0 |
| (13)Potassium hydroxide | 0.07 |
| (14)Phenoxyethanol | 0.5 |
| (15)Disodium edetate | 0.1 |

### Production method:

The powder components of (1) to (3) were mixed and then pulverized. A portion of the water phase of (10) to (13) was heated to 70 °C and the oil phase of (4), (5) and (8) heated to 70 °C was added thereto and emulsified to prepare an emulsified part. The powder components at room temperature were added and stirred into the remaining water phase at room temperature and the mixture was added and mixed to the emulsified part while the emulsified part was being stirred. Then, the liquid oil components of (6), (7) and (9) were gradually added and mixed into the mixture to obtain an emulsion eyeshadow. The viscosity of the emulsion eyeshadow was 7,000 mPa·s/30 °C. (peak temperature in the α-gel formation temperature range: 48 °C, temperature when the stirring for mixing was stopped: 38 °C)

### Example 10

| Emulsion eyeshadow | Blending quantity (mass%) |
|---|---|
| (1)Talc | 10.0 |
| (2)Kaolin | 2.0 |
| (3)Pigment | 5.0 |
| (4)Stearic acid | 1.0 |
| (5)Behenyl alcohol | 0.2 |
| (6)Isopropyl myristate | 6.0 |
| (7)Liquid paraffin | 5.0 |
| (8)Propylene glycol monolaurate | 3.0 |
| (9)Perfume | 0.05 |
| (10)Ion-exchanged water | balance |
| (11)Butylene glycol | 5.0 |
| (12)Glycerin | 1.0 |
| (13)Potassium hydroxide | 0.07 |
| (14)Phenoxyethanol | 0.5 |
| (15)Disodium edetate | 0.1 |

### Production method:

The powder components of (1) to (3) were mixed and then pulverized. A portion of the water phase of (10) to (13) was heated to 70 °C and the oil phase of (4) to (9) heated to 70 °C was added thereto and emulsified to prepare an emulsified part. The powder components at room temperature were added and stirred into the remaining water phase at room temperature and the obtained mixture was added and mixed to the emulsified part while the emulsified part was being stirred to obtain an emulsion eyeshadow. The viscosity of the emulsion eyeshadow was 7,000 sPa·s/30 °C. (peak temperature in the α-gel formation temperature range: 44 °C, temperature when the stirring for mixing was stopped: 39 °C)

### Example 11

| Hair cosmetic | Blending quantity (mass%) |
|---|---|
| (1)Liquid paraffin | 5.0 |
| (2)Petrolatum | 2.0 |
| (3)Dimethylpolysiloxane | 5.0 |
| (4)Cetanol | 4.0 |
| (5)Stearyl alcohol | 1.0 |
| (6)1,3-Butylene glycol | 10.0 |
| (7)Polyoxypropylene glyceryl ether | 2.0 |
| (8)Polyoxyethylene glyceryl isostearate | 2.0 |
| (9)Lipophilic glyceryl monostearate | 2.0 |
| (10)Polyquatemium-10 | 0.5 |
| (11)Purified water | balance |
| (12)Perfume | 0.1 |

### Production method:

The water phase (a portion of water phase) consisting of a portion of (6) and (11) (i.e., about 20 mass % of the total amount of (6) and (11)) was heated to 70 °C. The oil phase consisting of (2), (4), (7) and (8) to (9) was heated to 70 °C. The oil phase was gradually added to the water phase and emulsified with a homomixer to prepare an emulsified part. While the emulsified part was being stirred, the remaining water phase components (main water phase) of (6) and (11) at 25 °C, and the liquid oil components consisting of (1), (3), (10) and (12) were added and mixed, in order, into the emulsified part to obtain a hair cosmetic. The viscosity of the hair cosmetic was 4,000 mPa·s/30 °C. (peak temperature in the α-gel formation temperature range: 54°C, temperature when the stirring for mixing was stopped: 38 °C)

### Example 12

| Hair cosmetic | Blending quantity (mass%) |
|---|---|
| (1)Liquid paraffin | 5.0 |
| (2)Petrolatum | 2.0 |
| (3)Dimethylpolysiloxane | 5.0 |
| (4)Cetanol | 4.0 |
| (5)Stearyl alcohol | 1.0 |
| (6)1,3-Butylene glycol | 10.0 |
| (7)Polyoxypropylene glyceryl ether | 2.0 |
| (8)Polyoxyethylene glyceryl isostearate | 2.0 |
| (9)Lipophilic glyceryl monostearate | 2.0 |
| (10)Polyquaternium-10 | 0.5 |
| (11)Purified water | balance |
| (12)Perfume | 0.1 |

### Production method:

The water phase (a portion of water phase) consisting of a portion of (6) and (11) (i.e., about 20 mass % of the total amount of (6) and (11)) was heated to 70 °C. The oil phase consisting of (1) to (5), (7) to (9) and (12) was heated to 70 °C. The oil phase was gradually added to the water phase and emulsified with a homomixer to prepare an emulsified part. While the emulsified part was being stirred, the remaining water phase components (main water phase) at 25 °C were added and mixed into the emulsified part to obtain a hair cosmetic. The viscosity of the hair cosmetic was 4,000 mPa·s/30 °C. (peak temperature in the α-gel formation temperature range: 48 °C, temperature when the stirring for mixing was stopped: 42 °C)

### Example 13 (not according to the invention)

| Skin cleanser | Blending quantity (mass%) |
|---|---|
| (1)Ethanol | 15.0 |
| (2)Sorbitol solution | 10.0 |
| (3)Polyoxypropylene(9) diglyceryl ether | 4.0 |
| (4)Castor oil | 2.0 |
| (5)Isostearic acid | 2.0 |
| (6)Stearic acid | 7.0 |
| (7)Lauric acid | 6.0 |
| (8)Myristic acid | 11.0 |
| (9)Palmitic acid | 3.0 |
| (10)Sodium lauryl glycol carboxylate | 3.0 |
| (11)Sodium N-methyltaurate | 5.0 |
| (12)Sodium hydroxide | 4.0 |
| (13)Sodium chloride | 0.5 |
| (14)Chamomilla recutita extract | 0.1 |
| (15)Dibutyl hydroxytoluene | 0.1 |
| (16)Tetrasodium hydroxyethane diphosphonate (30%) | 0.1 |
| (17)Trisodium edetate | 0.1 |
| (18)4-tert-Butyl-4'-methoxydibenzoylmetane | 0.05 |
| (19)2-Ethylhexyl paramethoxycinnamate | 0.05 |
| (20)Mixture of sucrose and sorbitol | 15.0 |
| (21)Coloring agent | 0.5 |
| (22)Purified water | balance |
| (23)Perfume | 0.1 |

### Production method:

The water phase (a portion of water phase) consisting of a portion of (22) (i.e., about 20 mass % of the total amount of (22)), (12) and (13) was heated to 70 °C. The oil phase consisting of (3) and (5) to (11) was heated to 70 °C. The oil phase was gradually added to the water phase and emulsified with a homomixer to prepare an emulsified part. While the emulsified part was being stirred, the remaining water phase components (main water phase) of (1), (2), (14) to (17) and (20) to (22) at 25 °C, and the liquid oil components consisting of (4), (18), (19) and (23) were added and mixed, in order, into the emulsified part to obtain a skin cleanser. The viscosity of the skin cleanser was 2,500 mPa·s/30 °C. (peak temperature in the α-gel formation temperature range: 52 °C, temperature when the stirring for mixing was stopped: 41 °C)

### Example 14 (not according to the invention)

| Skin cleanser | Blending quantity (mass%) |
|---|---|
| (1)Ethanol | 15.0 |
| (2)Sorbitol solution | 10.0 |
| (3)Polyoxypropylene(9) diglyceryl ether | 4.0 |
| (4)Castor oil | 2.0 |
| (5)Isostearic acid | 2.0 |
| (6)Stearic acid | 7.0 |
| (7)Lauric acid | 6.0 |
| (8)Myristic acid | 11.0 |
| (9)Palmitic acid | 3.0 |
| (10)Sodium lauryl glycol carboxylate | 3.0 |
| (11)Sodium N-methyltaurate | 5.0 |
| (12)Sodium hydroxide | 4.0 |
| (13)Sodium chloride | 0.5 |
| (14)Chamomilla recutia extract | 0.1 |
| (15)Dibutyl hydroxytoluene | 0.1 |
| (16)Tetrasodium hydroxyethane diphosphonate (30%) | 0.1 |
| (17)Trisodium edetate | 0.1 |
| (18)4-tert-Butyl-4'-methoxydibenzoylmetane | 0.05 |
| (19)2-Ethylhexyl paramethoxycinnamate | 0.05 |
| (20)Mixture of sucrose and sorbitol | 15.0 |
| (21)Coloring agent | 0.5 |
| (22)Purified water | balance |
| (23)Perfume | 0.1 |

### Production method:

The water phase (a portion of water phase) consisting of a portion of (22) (i.e., about 40 mass % of the total amount of (22)), (12) and (13) was heated to 70 °C. The oil phase consisting of (3) to (11), (18), (19) and (23) was heated to 70 °C. The oil phase was gradually added to the water phase and emulsifed with a homomixer to prepare an emulsified part. While the emulsified part was being stirred, the remaining water phase components (main water phase) at 25 °C were added and mixed into the emulsifed part to obtain a skin cleanser. The viscosity of the skin cleanser was 2,500 mPa·s/30 °C. (peak temperature in the α-gel formation temperature range: 47 °C, temperature when the stirring for mixing was stopped: 43 °C)

## Claims

1. A production method of an O/W emulsion composition comprising the steps of:
emulsifying, at 70 ºC or higher, an oil phase with a portion of a water phase to prepare a high-temperature emulsified part that is a highly concentrated O/W emulsion, wherein
the oil phase comprises
(A) a nonionic surfactant,
(B) a linear higher alcohol that has 16 or more carbon atoms and can form an α-gel in water with the nonionic surfactant, and
(C) an oil component, and
the water phase comprises
(D) water;
mixing the remaining water phase, which is 50 to 75 mass % of the total amount of the O/W emulsion composition and at 10 to 35 ºC, with the high-temperature emulsified part while the high-temperature emulsified part is being stirred, to rapidly cool the high-temperature emulsified part with continuous stirring to the lower temperature limit, or lower, of a temperature range wherein the oil phase forms an α-gel in the water phase, wherein the lower temperature limit of the temperature range wherein the oil phase forms the α-gel in the water phase is 4 ºC lower than the peak temperature of the exothermic peak, which is measured by differential scanning calorimetry DSC, of the high-temperature emulsified part; and
then stopping the stirring.

2. A production method of an O/W emulsion composition, wherein the O/W emulsion composition comprises a liquid oil component and the method comprises the steps of:
emulsifying, at 70 ºC or higher, an oil phase with a portion of a water phase to prepare a high-temperature emulsified part that is a highly concentrated O/W emulsion, wherein
the oil phase comprises
(A) a nonionic surfactant,
(B) a linear higher alcohol that has 16 or more carbon atoms and can form an α-gel in water with the nonionic surfactant, and
(C) an oil component, and
the water phase comprises
(D) water,
wherein at least a portion of the liquid oil component is not contained in the oil phase;
mixing the remaining water phase, which is 50 to 75 mass % of the total amount of the O/W emulsion composition and at 10 to 35 ºC, with the high-temperature emulsified part while the high-temperature emulsified part is being stirred, to rapidly cool the high-temperature emulsified part with continuous stirring to the lower temperature limit, or lower, of a temperature range wherein the oil phase forms an α-gel in the water phase;
adding the remaining liquid oil component to the mixture at the lower temperature limit, or lower, of the α-gel formation temperature range; and
then stopping the stirring.

3. The method according to claim 2, wherein the liquid oil component in the oil phase for the preparation of the high-temperature emulsified part is less than 5 mass % of the total amount of the liquid oil component in the O/W emulsion composition.

4. The method according to claim 2 or 3, wherein the lower temperature limit of the temperature range wherein the oil phase forms the α-gel in the water phase is 8 ºC lower than the peak temperature of the exothermic peak, which is measured by differential scanning calorimetry DSC, of the high-temperature emulsified part.

## Patentansprüche

1. Verfahren zur Herstellung einer O/W-Emulsionszusammensetzung, umfassend die Schritte:
Emulgieren einer Ölphase bei 70°C oder höher mit einem Teil einer Wasserphase, um einen Hochtemperaturemulsionsteil zu erzeugen, bei welchem es sich um eine hochkonzentrierte O/W-Emulsion handelt, wobei
die Ölphase
(A) ein nicht-ionisches Tensid,
(B) einen linearen höheren Alkohol, welcher 16 oder mehr Kohlenstoffatome aufweist und in Wasser zusammen mit dem nicht-ionischen Tensid ein α-Gel ausbilden kann, und
(C) eine Ölkomponente umfasst, und
die Wasserphase
(D) Wasser umfasst;
Mischen der restlichen Wasserphase, welche 50 bis 75 Masse% der Gesamtmenge der O/W-Emulsionszusammensetzung ausmacht und auf 10 bis 35°C eingestellt ist, mit dem Hochtemperaturemulsionsteil unter gleichzeitigem Rühren des Hochtemperaturemulsionsteils, um den Hochtemperaturemulsionsteil unter kontinuierlichem Rühren rasch auf oder unter die Temperaturuntergrenze eines Temperaturbereichs abzukühlen, in welchem die Ölphase in der Wasserphase ein α-Gel ausbildet, wobei die Temperaturuntergrenze des Temperaturbereichs, in welchem die Ölphase in der Wasserphase das α-Gel ausbildet, 4°C unterhalb der Peaktemperatur des mittels Differentialscanningkalorimetrie DSC gemessenen exothermen Peaks des Hochtemperaturemulsionsteils liegt; und
anschließendes Beenden des Rührens.

2. Verfahren zur Herstellung einer O/W-Emulsionszusammensetzung, wobei die O/W-Emulsionszusammensetzung eine flüssige Ölkomponente umfasst und das Verfahren die Schritte umfasst:
Emulgieren einer Ölphase bei 70°C oder höher mit einem Teil einer Wasserphase, um einen Hochtemperaturemulsionsteil zu erzeugen, bei welchem es sich um eine hochkonzentrierte O/W-Emulsion handelt, wobei
die Ölphase
(A) ein nicht-ionisches Tensid,
(B) einen linearen höheren Alkohol, welcher 16 oder mehr Kohlenstoffatome aufweist und in Wasser zusammen mit dem nicht-ionischen Tensid ein α-Gel ausbilden kann, und
(C) eine Ölkomponente umfasst, und
die Wasserphase
(D) Wasser umfasst,
wobei zumindest ein Teil der flüssigen Ölkomponente nicht in der Ölphase enthalten ist;
Mischen der restlichen Wasserphase, welche 50 bis 75 Masse% der Gesamtmenge der O/W-Emulsionszusammensetzung ausmacht und auf 10 bis 35°C eingestellt ist, mit dem Hochtemperaturemulsionsteil unter gleichzeitigem Rühren des Hochtemperaturemulsionsteils, um den Hochtemperaturemulsionsteil unter kontinuierlichem Rühren rasch auf oder unter die Temperaturuntergrenze eines Temperaturbereichs abzukühlen, in welchem die Ölphase in der Wasserphase ein α-Gel ausbildet;
Hinzufügen der restlichen flüssigen Ölkomponente zu dem Gemisch bei oder unterhalb der Temperaturuntergrenze des zur Ausbildung von α-Gel befähigten Temperaturbereichs; und
anschließendes Beenden des Rührens.

3. Verfahren nach Anspruch 2, wobei die Menge an flüssiger Ölkomponente in der zur Herstellung des Hochtemperaturemulsionsteils fungierenden Ölphase weniger als 5 Masse% der Gesamtmenge an flüssiger Ölkomponente in der O/W-Emulsionszusammensetzung beträgt.

4. Verfahren nach Anspruch 2 oder 3, wobei die Temperaturuntergrenze des Temperaturbereichs, in welchem die Ölphase in der Wasserphase das α-Gel ausbildet, 8°C unterhalb der Peaktemperatur des mittels Differentialscanningkalorimetrie DSC gemessenen exothermen Peaks des Hochtemperaturemulsionsteils liegt.

## Revendications

1. Procédé de production d'une composition d'émulsion H/E, comprenant les étapes consistant :
à émulsifier, à 70 °C ou plus, une phase huileuse avec une partie d'une phase aqueuse pour préparer une partie émulsifiée à haute température qui est une émulsion H/E hautement concentrée, dans lequel
la phase huileuse comprend
(A) un tensioactif non ionique,
(B) un alcool supérieur linéaire qui a 16 atomes de carbone ou plus et peut former un α-gel dans l'eau avec le tensioactif non ionique, et
(C) un composant huileux, et
la phase aqueuse comprend
(D) de l'eau ;
à mélanger la phase aqueuse restante, qui représente 50 à 75 % en masse de la quantité totale de la composition d'émulsion H/E et à 10 à 35 °C, avec la partie émulsifiée à haute température pendant que la partie émulsifiée à haute température est sous agitation, pour refroidir rapidement la partie émulsifiée à haute température sous agitation continue à la limite de température inférieure, ou en dessous, d'une gamme de températures dans laquelle la phase huileuse forme un α-gel dans la phase aqueuse, et dans lequel la limite de température inférieure de la gamme de températures dans laquelle la phase huileuse forme l'α-gel dans la phase aqueuse est 4 °C inférieure à la température maximale du pic exothermique, qui est mesuré par calorimétrie différentielle à balayage DSC, de la partie émulsifiée à haute température ; et
puis à arrêter l'agitation.

2. Procédé de production d'une composition d'émulsion H/E, dans lequel la composition d'émulsion H/E comprend un composant huileux liquide et le procédé comprend les étapes consistant :
à émulsifier, à 70 °C ou plus, une phase huileuse avec une partie d'une phase aqueuse pour préparer une partie émulsifiée à haute température qui est une émulsion H/E hautement concentrée, dans lequel
la phase huileuse comprend
(A) un tensioactif non ionique,
(B) un alcool supérieur linéaire qui a 16 atomes de carbone ou plus et peut former un α-gel dans l'eau avec le tensioactif non ionique, et
(C) un composant huileux, et
la phase aqueuse comprend
(D) de l'eau ;
dans lequel au moins une partie du composant huileux liquide n'est pas contenue dans la phase huileuse ;
à mélanger la phase aqueuse restante, qui représente 50 à 75 % en masse de la quantité totale de la composition d'émulsion H/E et à 10 à 35 °C, avec la partie émulsifiée à haute température pendant que la partie émulsifiée à haute température est sous agitation, pour refroidir rapidement la partie émulsifiée à haute température sous agitation continue à la limite de température inférieure, ou en dessous, d'une gamme de températures dans laquelle la phase huileuse forme un α-gel dans la phase aqueuse ;
à ajouter le composant huileux liquide restant au mélange à la limite de température inférieure, ou en dessous, de la gamme de températures de formation de l'α-gel ; et
puis à arrêter l'agitation.

3. Procédé selon la revendication 2, dans lequel le composant huileux liquide dans la phase huileuse pour la préparation de la partie émulsifiée à haute température est inférieur à 5 % en masse de la quantité totale du composant huileux liquide dans la composition d'émulsion H/E.

4. Procédé selon la revendication 2 ou 3, dans lequel la limite de température inférieure de la gamme de températures dans laquelle la phase huileuse forme l'a-gel dans la phase aqueuse est 8 °C inférieure à la température maximale du pic exothermique, qui est mesuré par calorimétrie différentielle à balayage DSC, de la partie émulsifiée à haute température.
